(19) Europäisches Patentamt
European Patent Office
Office européen des brevets

(11) **EP 1 808 121 B1**

(12) **EUROPEAN PATENT SPECIFICATION**

(45) Date of publication and mention
of the grant of the patent:
**23.09.2009 Bulletin 2009/39**

(51) Int Cl.:
***A61B 5/02*** *(2006.01)*

(21) Application number: **06000458.7**

(22) Date of filing: **11.01.2006**

(54) **Method for measuring a user's cardiorespiratory endurance by a fitness equipment**

Verfahren zur Messung der kardio-respiratorischen Ausdauer einer Versuchsperson mittels eines Fitness-Gerätes

Méthode pour le mesurage de l'endurance cardio-respiratoire d'une personne par un dispositf d'entraînement

(84) Designated Contracting States:
**DE ES FR GB IT SE**

(43) Date of publication of application:
**18.07.2007 Bulletin 2007/29**

(73) Proprietor: **Wang, Leao**
**Taiping City,**
**Taichung Hsien, 411**
**Taiwan R.O.C. (TW)**

(72) Inventor: **Wang, Leao**
**Taiping City,**
**Taichung Hsien, 411**
**Taiwan R.O.C. (TW)**

(74) Representative: **Reichel, Wolfgang et al**
**Reichel und Reichel Patentanwälte**
**Parkstrasse 13**
**60322 Frankfurt am Main (DE)**

(56) References cited:
**US-A- 4 112 928        US-A- 4 566 461**
**US-A- 5 769 755**

**Description**

The invention relates to a method for measuring a user's cardiorespiratory endurance by a fitness equipment, and more particularly to a method for obtaining a reference value of the user's cardiorespiratory endurance by a general fitness equipment having a specific function, such that users can know about their physical conditions or use the reference value as a basis for further physical training.

As we all know, the cardiorespiratory endurance is a major factor for determining our physical conditions, and thus knowing about one's cardiorespiratory functions and conditions by a popular fitness equipment and a simple procedure definitely can alert us about our health conditions and remind us to improve our physical conditions and further maintain our health. Similarly, the physical conditions absolutely affect the Overall performance of a professional athletic, and thus professional athletics are trained to improve their cardiorespiratory endurance, which such training is an important part for maintaining their professional performance.

However, up to now, there is no fitness equipment having the foregoing function of measuring our cardiorespiratory endurance in a simple manner. Therefore, people regardless of the general public or professional athletics have to go to hospitals, related research institutes, or professional sport physiological laboratories for the physical examinations using expensive cardiorespiratory testing devices or related equipments. In addition to the costs, such arrangement also causes inconvenience to users.

As to most users, they maintain their physical strength and functions by a so-called "progressive self training" and determine whether or not their physical strength and functions make improvements or breakthroughs by their subjective recognition and self-conscience. However, such subjective inspection is simply a conscious evaluation without strong supports by scientific data, and thus easily causing distortion or discrepancy. Since our cardiorespiratory endurance varies with many subjective and objective factors (such as the level of the examinee's physiological tiredness, psychological conditions, external environment, and climatic factors, etc). If there is no definite support of scientific data, the testing results will be questionable, and people will lose their benefits of having positive selftraining.

Therefore, it is a primary object of the invention to provide an existing fitness equipment having a specific operating functions (such as the model that comes with a speed control meter and a built-in program for entering the user's age, and automatically calculating the optimal heartbeat rate, basic exercise safe heartbeat rate, and a heartbeat monitor) to operate with a simple mid-strength testing exercise procedure for effectively obtaining the best reliable reference value of the cardiorespiratory endurance, and further effectively maximizing the utility, value, and significance of the fitness equipment. To each user, regardless of professional athletics or general public, such fitness equipment can be used to provide the information about the user's physical function and conditions and assure the user's health conditions, so as to maintain and improve our cardiorespiratory endurance.

A heart rate monitor device for use in monitoring aerobic exercises training is known from the US-A-4,566,461. The maximal oxygen uptake capacity is calculated and is displayed as a fitness parameter. This document describes a monitoring device as prior art which is performed so that at the end of a twelve minute running period the fitness parameter is derived from a simple look-up table based on the distance covered in the twelve minutes. According to a referred embodiment of the document a heart rate is measured by a jogging-computer. One exercise comprises a slow rate period of three minutes and at faster cadences of three minutes. The maximal heart rate is calculated at 200 minus age. The exercises are performed with a maximal oxygen uptake of 60 - 80%. The maximal oxygen uptake (% $VO_{2max}$) is calculated at values of a rest heart rate $HR_R$ and the maximal heart rate $HR_M$ by the formula:

$$\% \ VO_{2max} \ = \ \frac{HR \ - \ HR_R + 10}{HR_M \ - \ HR_R}$$

Another solution for measuring a fitness parameter using an ergometer is disclosed in the US-A-4,112,928. By a computer the oxygen absorption capacity can be indicated. This device use the sum of the energy indicated as calories received by the ergometer to indicated the fitness whereby the time of the training and the intensity are determined as a basis.

Therefore, the present invention mainly uses a fitness equipment such as an electric treadmill having a speed control meter panel and a heartbeat monitor to operate with the optimal human heartbeat rate (which is 60% - 90% of our maximum heartbeat rate), the basic exercise safe heartbeat rate (which is 40% - 60% of our maximum heartbeat rate) and a predetermined mid-strength testing exercise procedure for easily measuring the reference value of the cardiorespiratory endurance (also known as cardiorespiratory function) of a user at the time of the measurement, so as to provide the user a reference of physical conditions and a basis for future physical training and exercise.

The accomplishment of this and other objects of the invention will become apparent from the following description and its accompanying drawings of which:

FIG. 1     is a flow chart of a testing exercise procedure according to a preferred embodiment of the present invention;

FIG. 2     is a list of measuring results of a first examinee according to a preferred embodiment of the present invention; and

FIG. 3     is a list of measuring results of a second examinee according to a preferred embodiment of the present invention.

Before the testing method in accordance with the present invention is described, some of the terminologies used in this specification are explained first.

Maximum Heartbeat Rate: It refers to the maximum heartbeat that a human body can "possibly" bear. At present, both medical and academic areas accept 220 times per minute as the maximum heartbeat rate.

Optimal Heartbeat Rate: It is equal to the maximum heartbeat rate subtracted by the examinee's age and then multiplied by 60% - 90%. At present, both medical and academic areas accept such reference value as the optimal heartbeat rate.

Basic Exercise Safe Heartbeat Rate: It is equal to the maximum heartbeat rate subtracted by the examinee's age and then multiplied by 40% - 60%. At present, both medical and academic areas accept such reference value as the optimal heartbeat rate.

Monitoring Meter Panel: It is an electronic meter panel for monitoring the exercise speed, distance, and related parameters of a fitness equipment, and capable of displaying the parameters on a specific window of the panel. Most of the present fitness equipments come with this device.

Heartbeat Monitor: It is a device being in contact with an examinee for detecting the heartbeats of the examinee anytime, and capable of display the heartbeats on a specific window of the speed control meter panel. Most of the present fitness equipments come with this device.

Cardiorespiratory Endurance Value (abbreviated as RSV HRC): The RSV HRC value finally measured by the method of the present invention is based on the optimal heartbeat rate as defined above (and the optimal heartbeat rate is used as the continuous load limit).

Testing Exercise: It could be the jogging exercise on a treadmill, pedaling exercise on a fitness bike, or simulated running exercise of an elliptical trainer, depending on the type of the fitness equipment.

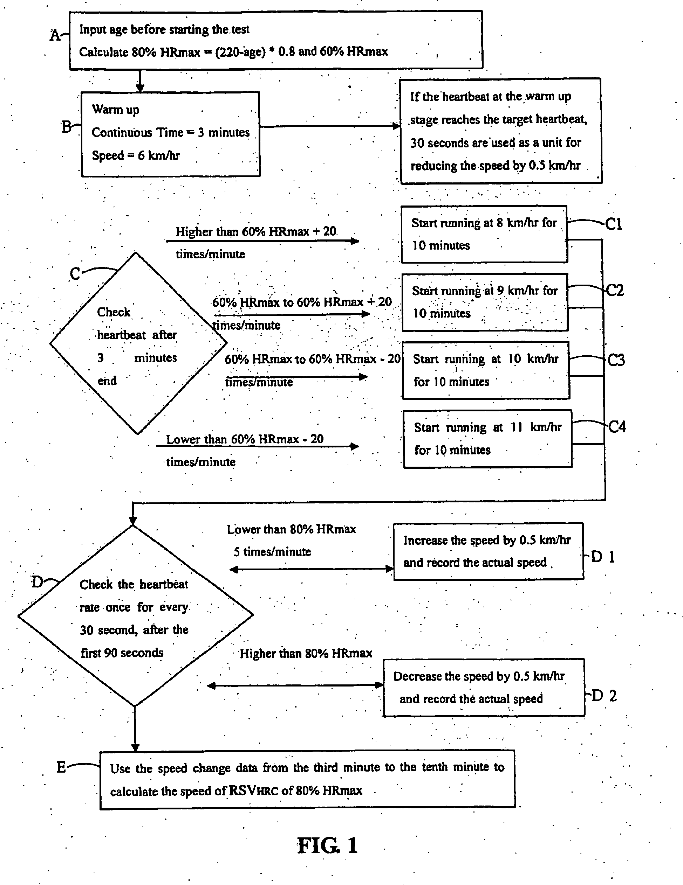

Now, referring to FIG. 1 for the flow chart of the testing exercise procedure according to a preferred embodiment of the present invention, this preferred embodiment adopts 80% of the maximum heartbeat rate subtracted by the examinee's age as the optimal heartbeat rate, and 60% of the maximum heartbeat rate subtracted by the examinee's age as the basic exercise safe heartbeat, and 10 minutes of the exercise time as the basis of the testing conditions, and the procedure comprises the following steps:

Step A: Enter the user's age into the monitoring meter panel of a fitness equipment, and a built-in program of the monitoring meter panel calculates the user's 80% as the optimal heartbeat rate value and 60% as the basic exercise safe heartbeat rate value;

Step B: Allow the user to warm up continuously for three minutes, and set the exercise speed to 6 Km/hr, and progressively lower the exercise speed if the user's heartbeat value is too large;

Step C: Check the heartbeat value;

Step C 1: Start a mid-strength testing exercise at the speed of 8 Km/hr for 10 minutes, if the measured heartbeat value is higher than the sum of the basic exercise safe heartbeat rate value and 20 times/minute;

Step C2: Start a mid-strength testing exercise at the speed of 9 Km/hr for 10 minutes, if the measured heartbeat value is between the basic exercise safe heartbeat rate value and the sum of the basic exercise safe heartbeat rate value and 20 times/minute;

Step C3: Start a mid-strength testing exercise at the speed of 10 Km/hr for 10 minutes, if the measured heartbeat value is between the basic exercise safe heartbeat rate value and the difference of the basic exercise safe heartbeat rate value and 20 times/minute;

Step C4: Start a mid-strength testing exercise at the speed of 11 Km/hr for 10 minutes, if the measured heartbeat value is lower than the difference of the basic exercise safe heartbeat rate value and 20 times/minute;

Step D: From the first 90 seconds on, check the heartbeat value once for every 30 seconds and record the heartbeat value;

Step D1: Increase the speed by 0.5Km/hr if the measured heartbeat value is 5 times/minute lower than the optimal heartbeat value, and record the speed;

Step D2: Decrease the speed by 0.5Km/hr if the measured heartbeat value is higher than the optimal heartbeat value, and record the speed;

Step E: Complete the 10-minute testing exercise and substitute the actual speed reading from the third minute to the tenth minute into the following formula to obtain the RSV HRC value. The formula for calculating RSV HRC value is given below:

$$\text{RSV HRC} = \frac{\Sigma(d-Md) \times (T-MT)}{\Sigma(T-MT)^2}$$

where

| | |
|---|---|
| d-Md | is an average difference between the exercise distances; |
| T-MT | is the average difference between the exercise time; and |
| $\Sigma$ | is the sum. |

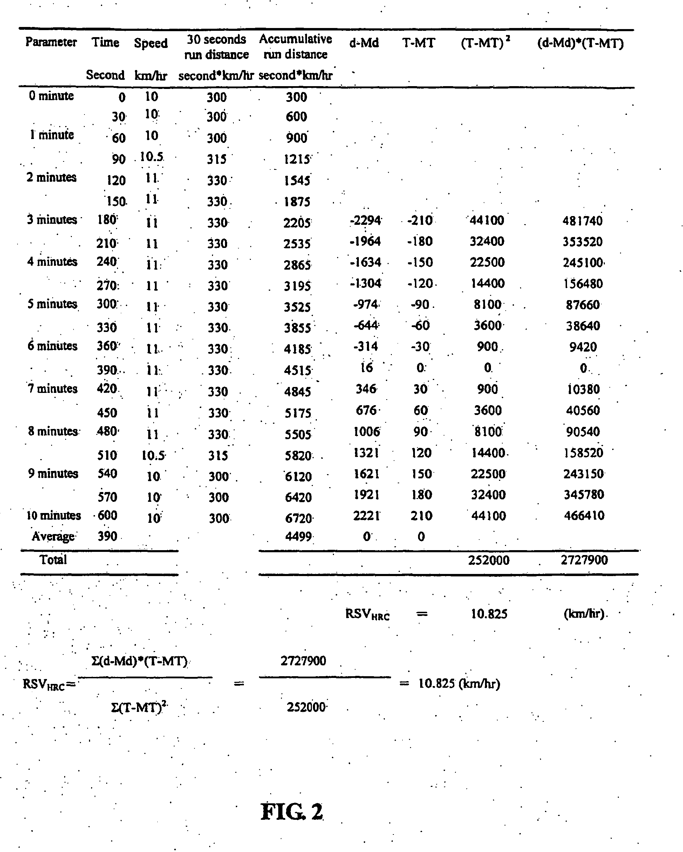

Referring to FIG. 2 for the list of measuring results of a first examinee (who uses an electric treadmill as the testing model), the examiner can have a better understanding about the operation of the formula. The average time used by the first examinee is 390 (which is a constant), and the average exercise distance is 4499 (which is usually a variable). Therefore, the T-MT value at the third minute is equal to 80-390 = -210; the d-Md value is equal to 2205-4499 = -2294; and so on, and (T-MT)$^2$ is equal to -210x-210 = 44100, and (d-Md)x(T-MT) is equal to -2294x-210 = 481740.
The RSV HRC value equals to the distance difference multiplied by the sum of time differences and divided by the sum of squares of the time differences, as follows:

$$2727900-252000=10.825(km/hr)$$

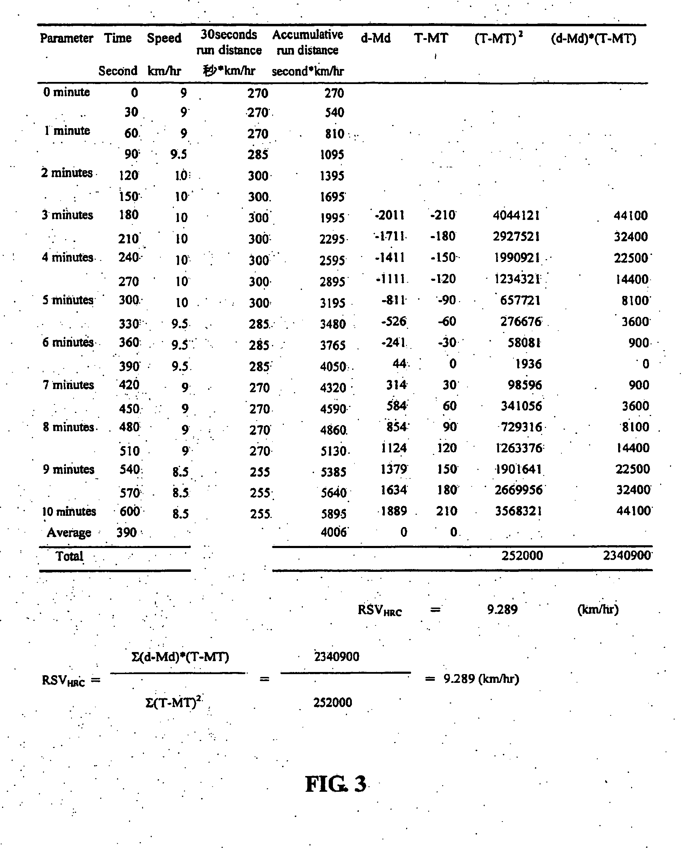

Referring to FIG. 3 for the list of measuring results of a second examinee compared with the list of measuring results of the first examinee shown in FIG. 2, the higher the examinee's RSV HRC value, the better is the examinee's cardiorespiratory endurance. In other words, the exercise distance becomes larger and the exercise speed becomes faster per same testing unit time. The lower the heartbeat value, the better is the cardiorespiratory function. This testing method has been used for testing several tens of examinees to obtain the actual test results, and the test results showed that this method has high reliability and it is definitely a practical and useful testing method.
The inventor of the present invention adopted the so-called "Basic Exercise Safe Heartbeat Rate" as the standard for measuring the heartbeat and constant exercise speed after the warm up, because the heartbeat value will increase and accelerate according to the level of exercise when a person is doing an aerobic exercise. Based on the safety factor, the present invention designs a "Safety Heartbeat Standard" to prevent an overload of the examinee's heartbeat. More particularly, such arrangement provides a protection for people whose cardiorespiratory function have been damaged seriously, elderly people, or people having a poor cardiorespiratory function. Let us take an examinee of age 20 and an examinee of age 60 for examples as follows:

$$(220-20) \times 60\% = 120 \text{times/minute}$$

$$(220-60) \times 60\% = 96 \text{times/minute}$$

From the above expressions, examinees of different ages have a small difference between their basic exercise safe heartbeat rate values, and thus the present invention uses such difference as the standard for starting the measurements of heartbeats and constant exercise speeds. Further, the calculation of using positive and negative 20 times/minute is adopted to accurately distinguish the examinees with a special physical capability (either having a very good physical capability or having a very poor physical capability) to obtain a safer and gentler testing procedure.

Further, the so-called "Mid-Strength Exercise" refers to a "Non-Exhausted Exercise" used for measuring the cardiorespiratory endurance reference value, primarily emphasizing on making this method applicable for each people who uses the fitness equipment, regardless of male, female, elderly, or youth. As described above, a more precise professional equipment or device is needed for measuring the actual cardiorespiratory endurance value of each examinee, and the examinee has to try very hard to exercise until his/her strength is exhausted before an accurate cardiorespiratory value can be obtained. Further, the same testing procedure must be repeated several times to obtain a more definite and truer testing data. However, most of the examinees do not have to go through such a violent and potentially dangerous testing procedure, but just want to have a general idea about their current physical conditions by using the fitness equipment. Furthermore, examinees should not do excessively violent exercises without being accompanied by professional trainers or medical personnel. Therefore, the testing procedure and a gentler mid-strength exercise in accordance with the present invention can assure the safety of the examinees.

With the same testing procedure and RSV HRC formula, the present invention operates with different optimal heartbeat rate values, basic exercise safe heartbeat rate values, and exercise time (such as the standard of using 90% for the optimal heartbeat rate, 50% for the basic exercise safe heartbeat rate, and 15 minutes for the exercise time) to measure the RSV HRC reference value. However, the data or parameters used as the measuring standard for obtaining the best testing reference value relies on our actual practices, experiments, and verifications.

Obviously, these standards will not affect the validity, usefulness, and convenience of the present invention.

**Claims**

1. A method for measuring a user's cardiorespiratory endurance by a fitness equipment, comprising the step of using a fitness equipment having a speed control meter panel and a heartbeat monitor to operate with an optimal heartbeat rate, a basic exercise safe heartbeat rate, and a predetermined mid-strength testing exercise procedure for easily measuring said user's cardiorespiratory reference value (RSV HRC) at the time of measurement, **characterise in that**, said RSV HRC value is calculated by the formula:

$$\text{RSV HRC} = \frac{\Sigma(d-Md) \times (T-MT)}{\Sigma(T-MT)^2}$$

where d-Md is equal to an average difference of exercise distances;
T-MT is equal to an average difference of exercise time; and
$\Sigma$ is the sigma for a sum.

2. The method of measuring a user's cardiorespiratory endurance by a fitness equipment of claim 1, wherein said optimal heartbeat rate is defined as the heartbeat rate falling in the range of 60% - 90% of a maximum heartbeat rate.

3. The method of measuring a user's cardiorespiratory endurance by a fitness equipment of claim 1, wherein said basic exercise safe heartbeat rate is defined as the heartbeat rate falling in the range of 40% - 60°s of the maximum heartbeat rate.

4. The method of measuring a user's cardiorespiratory endurance by a fitness equipment of claim 2 and 3, wherein a maximum heartbeat rate is equal to said maximum heartbeat rate subtracted by the examinee's age.

5. The method for measuring a user's cardiorespiratory endurance by a fitness equipment of one of the preceding claims, comprising the step of:

   (A) entering the user's age into a monitor meter panel, and a built-in program of said monitor meter panel calculating a user's predetermined optimal heartbeat rate value and a predetermined basic exercise safe heartbeat rate value;

(B) allowing said user to warm up continuously for at least a minimum warm up phase time, and setting an exercise speed, and progressively reducing said exercise speed if the heartbeat value of said user is too large;
(C) checking said heartbeat value;

(CI): starting a mid-strength testing exercise at a predefined speed $v_1$ for a predefined exercise time $t_{ex}$, if the measured heartbeat value is larger than the sum of said basic exercise safe heartbeat value and a predefined tolerance heartbeat value;
(C2) starting a mid-strength testing exercise at a predefined speed $v_2$ for said predefined exercise time $t_{ex}$, if the measured heartbeat value is between said basic exercise safe heartbeat value and the sum of said basic exercise safe heartbeat value and said predefined tolerance heartbeat value; (C3) starting a mid-strength testing exercise at a predefined speed $v_3$ for said predefined exercise time $t_{ex}$, if the measured heartbeat value is between said basic exercise safe heartbeat value and the difference of said basic exercise safe heartbeat value and said predefined tolerance heartbeat value; (C4) starting a mid-strength testing exercise at a predefined speed $v_9$ for said predefined exercise time $t_{ex}$, if the measured heartbeat value is lower than the difference of said basic exercise safe heartbeat value and said predefined tolerance heartbeat value;

(D) examining a heartbeat value in predefined time steps starting after a predefined time, and recording said heartbeat value;

(D1) increasing said exercise speed for a predefined speed amount, if said measured heartbeat value is lower than said optimal heartbeat value by a predefined heartbeat rate, and recording said exercise speed;,
(D2) decreasing said exercise speed for said predefined speed amount, if said measured heartbeat value is higher than said optimal heartbeat value, and recording said exercise speed;

(E) completing said testing exercise in a predetermined unit time, and inputting actual exercise speeds measured from a predefined point in time to the end of said exercise into a formula to obtain said RSV HRC value.

6. The method of measuring a user's cardioxespiratory endurance by a fitness equipment of claim 6, wherein said exercise speed in step (B) being set to 6 Km/hr, said minimum warm up phase time in step (B) being three minutes, said predefined speed $v_1$ in step (C1) being at least 8 Km/hr, said predefined speed $v_2$ in step (C2) being at least 9 Km/hr, said predefined speed $v_3$ in step (C3) being at least 10 Km/hr, said predefined speed $v_9$ in step (C4) being at least 11 Km/hr, said predefined exercise time $t_{ex}$ in steps (C1) to (C4) being at least ten minutes, said predefined tolerance heartbeat value in steps (C1) to (C4) being 20 times/minute, the predefined time steps in step (D) being every 30 seconds, the predefined time in step (D) being 90 seconds, the predefined speed amount in steps (D1) and (D2) being at least 0.5 Km/hr, the predefined heartbeat rate in step (D1) being 5 times/minute and the predefined point in time in step (E) being the third minute.

**Patentansprüche**

1. Ein Verfahren zum Messen einer kardiorespiratorischen Ausdauer eines Benutzers durch ein Fitness- Gerät, umfassend den Schritt des Verwendens eines Fitness- Geräts, das ein Geschwindigkeitskontrollmessinstrumentenbrett und einen Herzschlag- Monitor aufweist, um mit einer optimalen Herzschlag- Rate zu arbeiten, eine Basisübungssicherheits-Herzschlagrate und eine vorbestimmte mittlere Belastungstest- Übungsprozedur, um einfach den besagten kardiorespiratorischen Referenzwert (RSV HRC) zum Zeitpunkt der Messung zu messen, **dadurch gekennzeichnet, dass** der besagte RSV HRC- Wert durch folgende Formel berechnet wird:

$$RSV\ HRC = \frac{S\ (d\text{-}Md)\ x\ (T\text{-}MT)}{S\ (R\text{-}MT)^2}$$

wobei d- Md gleich zu einer durchschnittlichen Differenz einer Ühungsdistanz ist;
T- MT ist gleich zu einer durchschnittlichen Differenz einer Ausführungszeit; und
S ist das Sigma für eine Summe.

2. Das Verfahren zum Messen der kardiorespiratorisehen Ausdauer eines Benutzers durch ein Fitness- Gerät nach Anspruch 1, wobei die besagte optimale Herzschlagrate definiert ist als die Herzschlagrate, die in dem Bereich von 60% - 90% einer Maximum-Herzschlagrate fällt.

3. Das Verfahren zum Messen einer kardiorespiratorischen Ausdauer eines Benutzers durch ein Fitness- Gerät nach Anspruch 1, wobei die besagte Basisübungssiclierlieits-Herzschlagrate definiert ist als die Herzschlagrate, die in dem Bereich von 40% - 60% der Maximum- Herzschlagrate fällt.

4. Das Verfahren zum Messen einer kardiorespiratorischen Ausdauer eines Benutzers durch ein Fitness- Gerät nach Anspruch 2 und 3, wobei eine Maximum- Herzschlagrate gleich zu der besagten Maximum- Herzschlagrate ist, subtrahiert mit dem Alter des Benutzers.

5. Das Verfahren zum Messer einer kardiorespiratorischen Ausdauer durch ein Fitness-Gerät nach einem der vorhergehenden Ansprüche, umfassend die Schritte:

(A) Eingeben des Alters des Benutzers in ein Monitormeter- Instrumentenbrett und ein eingebautes Programm des besagten Monitormeter- Instrumentenbrettes berechnet, den vorbestimmten optimalen Herzschlagwert eines Benutzers und bestimmt den Basisübungssicherheits- Herzschlagsratenwert;
(B) Dem Benutzer erlauben, sich kontinuierlich für zumindest eine Minimum- Aufwärm-Phasenzeit aufzuwärmen, Setzen einer Übungsgeschwindigkeit, und progressives Reduzieren der besagten Übungsgeschwindigkeit, wenn der Herzschlagwert des besagten Benutzers zu hoch ist;
(C) Überprüfen des besagten Herzschiagswertes;

(C1): Starten einer Testübung mittlerer Stärke in einer vordefinierten Geschwindigkeit $v_1$ für eine vordefinierte Übungszeit $t_{ex}$, falls der gemessene Herzschlagswert größer ist als die Summe des besagten Basisübungssicherheits- Herzschlagswerts und eines vordefinierten Toleranz- Herzschlagswertes;
(C2) Starten einer Testübung in mittlerer Stärke in einer vordefinierten Geschwindigkeit $V_2$ für die besagte vordefinierte Übungszeit $t_{ex}$, falls der gemessene Herzschlagswert zwischen dem besagten Basisübungs-Sicherheitsherzschlagswert und der Summe des besagten Basisübungssicberbeits- Herzschlagswerts und des besagten vordefinierten Toleranz- Herzschlagswertes; (C3) ist, Starten einer Testübung mit mittlerer Stärke in einer vordefinierten Geschwindigkeit $v_3$ für die besagte Übungszeit $t_{ex}$, falls der gemessene Herzschlagswert zwischen dem besagten Basisübungssicherheits-Herzschlagwert und der Differenz des besagten Basisübungs- Sicherlieitsherzschlagwertes und des besagten vordefinierten Toleranz- Herzschlagswertes ist; (C4) Starten einer Testübung in mittlerer Stärke in einer vordefinierten Geschwindigkeit $v_9$ für die besagte vordefinierte Ausübungszeit $t_{ex}$, falls der gemessene Herzschlagswert geringer als die Differenz des besagten Basisübungs- Sicherheitsherrschlagswert und des besagten vordefinierten Toleranz-Herzschlagswertes ist;

(D) Prüfen eines Herzschlagwertes in vordefinierten Zeitschritten, startend nach einer vordefinierten Zeit und Aufzeichnen des besagten Herzschlagswertes;

(D1) Erhöhen der besagten Ausübungsgeschwindigkeit für eine vordefinierte Geschwindigkeitsgröße, falls der gemessene Herzschlagswert um eine vordefinierten Herzschlagsrate kleiner als der besagte optimale Herzschlagwert ist und Aufzeichnen der besagten Ausühungsgeschwindigkeit;
(D2) Heruntersetzen der Übungsgcscliwindigkeit um die besagten vordefinierten Gescliwindigkcitsgröße, falls besagter gemessener Herzschlagswert höher als der besagte optimale Herzschlagswert ist, und Aufzeichnen der Übungsgeschwindigkeit;

(E) Beenden der besagten Testübungen in einer vorbestimmten Zeiteinheit und Eingeben der aktuellen Übungsgeschwindigkeiten, gemessen von einem vordefinierten Zeitpunkt bis zum Ende der besagten Übung in eine Formel, um besagten RSV HRC- Wert zu erlangen.

6. Das Verfahren zum Messen einer kardiorespiratorischen Ausdauer eines Benutzers durch ein Fitness- Gerät nach Anspruch 5, wobei die besagte Übungsgeschwindigkeit in Schritt (B) auf 6km/Std. gesetzt wird, die besagte Minimum-Aufwärmzeitphase in Schritt (B) wird auf drei Minuten gesetzt, die besagte vordefinierte Geschwindigkeit $V_1$ in Schritt (C1) ist zumindest 8km/Std., die vorbestimmte Zeit V2 in Schritt (C2) ist zumindest 9km/Std., die vorbestimmte Geschwindigkeit V3 in Schritt (C3) ist zumindest 10km/Std., die vordefinierte Geschwindigkeit V9 in Schritt (C4) ist zumindest 11km/Std., die vorbestimmte Übungszeit $t_{ex}$ in den Schritten (C1) bis (C4) ist zumindest 10 Minuten, der vorbestimmte Toleranz- Herzschlagwert in Schritten (C1) bis (C4) ist 20 mal/Minute, die vordefinierte Zeitschritte in Schritt (D) sind alle 30 Sekunden, der vordefinierte Zeitschritt in (D) ist 90 Sekunden, die vordefinierte Geschwindigkeitshöhe in Schritten (D1) und (D2) sind zumindest 0,5 km/Std., die vordefinierte Herzschlagsrate in Schritt (D1) ist zumindest 5 mal/Minute und der vordefinierten Zeitpunkt in Schritt (E) ist die dritte Minute.

**Revendications**

1. Une méthode de mesure de l'endurance cardio-respiratoire d'une personne par un dispositif d'entraînement, comportant l'étape d'utiliser un dispositif d'entraînement doté d'un panneau de mesure et contrôle de vitesse et un dispositif de contrôle des battements cardiaques à faire fonctionner à un rythme cardiaque optimal, un rythme cardiaque de sécurité pour un exercice de base, et une procédure prédéterminée d'exercice d'essai à mi-puissance pour mesurer facilement la valeur de référence cardio-respiratoire (RSV HRC) de ladite personne au moment de la mesure, **caractérisée en ce que** ladite valeur RSV HRC est calculée suivant la formule:

$$RSV\ HRC = \frac{\Sigma(d-Md)\times(T-MT)}{\Sigma(T-MT)^2}$$

Avec d-Md est égale à une différence moyenne des distances d'exercice;
T-MT est égal à une différence moyenne des temps d'exercice; et
$\Sigma$ est le sigma pour une somme.

2. La méthode de mesure de l'endurance cardio-respiratoire d'une personne par un dispositif d'entraînement selon la revendication 1, dans laquelle ledit rythme cardiaque optimal est défini comme étant le rythme cardiaque se situant dans une gamme de 60% à 90% du rythme cardiaque maximum

3. La méthode de mesure de l'endurance cardio-respiratoire d'une personne par un dispositif d'entraînement selon la revendication 1, dans laquelle ledit rythme cardiaque de sécurité d'un d'exercice de base est défini comme étant le rythme cardiaque se situant dans une gamme de 40% à 60% du rythme cardiaque maximum.

4. La méthode de mesure de l'endurance cardio-respiratoire d'une personne par un dispositif d'entraînement selon les revendications 2 et 3, dans laquelle le rythme cardiaque maximum est égal audit rythme cardiaque maximum auquel on soustrait l'âge de la personne examinée..

5. La méthode de mesure de l'endurance cardio-respiratoire d'une personne par un dispositif d'entraînement selon l'une des revendications précédentes, comportant les étapes de :

(A) entrer l'âge de la personne au moyen d'un panneau de mesure et de contrôle, et un programme intégré audit panneau de mesure et de contrôle calculant une valeur prédéterminée du rythme cardiaque optimal et une valeur prédéterminée du rythme cardiaque de sécurité pour un exercice de base
(B) laisser la personne effectuer un échauffement continu pendant une durée d'échauffement minimum, fixer une vitesse d'exercice et progressivement réduire ladite vitesse d'exercice si le rythme cardiaque de la personne est trop élevée ;
(C) vérifier la valeur du rythme cardiaque ;

(C1) commencer un exercice d'essai à mi-puissance à une vitesse prédéfinie $v_1$, pour un temps d'exercice prédéfini $t_{ex}$, si la valeur du rythme cardiaque mesurée est plus grande que la somme de la valeur du rythme cardiaque de sécurité pour un exercice de base et d'une valeur prédéfinie du rythme cardiaque de tolérance;
(C2) commencer un exercice d'essai à mi-puissance à une vitesse prédéfinie $v_2$ pour ledit temps d'exercice prédéfini $t_{ex}$, si la valeur du rythme cardiaque mesurée se situe entre ladite valeur du rythme cardiaque de sécurité pour un exercice de base et la somme de ladite valeur du rythme cardiaque de sécurité pour un exercice de base et ladite valeur prédéfinie de tolérance du rythme cardiaque;
(C3) commencer un exercice d'essai à mi-puissance à une vitesse prédéfinie $v_3$ pour ledit temps d'exercice prédéfini $t_{ex}$, si la valeur du rythme cardiaque mesurée se situe entre ladite valeur du rythme cardiaque de sécurité pour un exercise de base et la différence de la valeur du rythme cardiaque de sécurité pour un exercice de base avec la valeur prédéfinie du rythme cardiaque de tolérance;
(C4) commencer un exercice d'essai à mi-puissance à une vitesse prédéfinie $v_9$ pour ledit temps d'exercice prédéfini $t_{ex}$, si la valeur du rythme cardiaque mesurée est inférieure à la différence entre la valeur du rythme cardiaque de sécurité pour un exercice de base et la valeur prédéfinie du rythme cardiaque de tolérance ;

(D) examiner la valeur du rythme cardiaque à des étapes prédéfinies commençant après un temps prédéfini, et enregistrer ladite valeur du rythme cardiaque ;

(D1) augmenter ladite vitesse d'exercice d'une quantité prédéfinie, si ladite valeur du rythme cardiaque mesurée est inférieure à ladite valeur optimale du rythme cardiaque d'un montant prédéfini, et enregistrer ladite vitesse d' exercice;
(D2) réduire ladite vitesse d' exercice dudit montant prédéfini, si la valeur du rythme cardiaque mesurée est plus élevée que la valeur du rythme cardiaque optimal, et enregistrer ladite vitesse d' exercice ;

(E) exécuter ledit exercice d' essai dans un temps prédéterminé, et intégrer les vitesses réelles d'exercice mesurées à partir d'un instant prédéfini jusqu'à la fin dudit exercice au sein d'une formule permettant d'obtenir la valeur dite de RSV HRC.

**6.** La méthode de mesure de l'endurance cardio-respiratoire d'une personne par un dispositif d'entraînement suivant la revendication 5, dans laquelle la vitesse d'exercice durant l'étape (B) est fixée à 6 Km/hr, la durée de la phase minimale d'échauffage dans la phase (B) étant fixée à trois minutes, ladite vitesse prédéfinie $v_1$ de l'étape (C1) étant au moins 8Km/hr, ladite vitesse prédéfinie $v_2$ de l'étape (C2) étant fixée à au moins 9 Km/hr, ladite vitesse prédéfinie $v_3$ de l'étape (C3) étant fixée à au moins 10 Km/hr, ladite vitesse prédéfinie $v_9$ dans l'étape (C4) étant fixée à au moins 11 Km/hr, ledits temps d'exercice prédéfinis $t_{ex}$ dans les étapes (C1) à (C4) étant au moins de dix minutes, ladite valeur prédéfinie du rythme cardiaque de tolérance dans les étapes (C1) à (C4) étant de 20 fois/minute, les délais prédéfinis dans l'étape (D) étant toutes les 30 secondes, le temps prédéfini dans l'étape (D) étant de 90 secondes, la quantité de vitesse prédéfinie dans les étapes (D1) et (D2) étant au moins 0.5 Km/hr, le rythme cardiaque prédéfini dans l'étape (D1) étant 5 fois/minute et la point prédéfini dans l'étape (E) étant la troisième minute.

A — Input age before starting the test

Calculate 80% HRmax = (220-age) * 0.8 and 60% HRmax

B — Warm up
Continuous Time = 3 minutes
Speed = 6 km/hr

If the heartbeat at the warm up stage reaches the target heartbeat, 30 seconds are used as a unit for reducing the speed by 0.5 km/hr

Higher than 60% HRmax + 20 times/minute

Start running at 8 km/hr for 10 minutes ——C1

C — Check heartbeat after 3 minutes end

60% HRmax to 60% HRmax + 20 times/minute

Start running at 9 km/hr for 10 minutes ——C2

60% HRmax to 60% HRmax - 20 times/minute

Start running at 10 km/hr for 10 minutes ——C3

Lower than 60% HRmax - 20 times/minute

Start running at 11 km/hr for 10 minutes ——C4

Lower than 80% HRmax 5 times/minute

Increase the speed by 0.5 km/hr and record the actual speed — D 1

D — Check the heartbeat rate once for every 30 second, after the first 90 seconds

Higher than 80% HRmax

Decrease the speed by 0.5 km/hr and record the actual speed — D 2

E — Use the speed change data from the third minute to the tenth minute to calculate the speed of RSVHRC of 80% HRmax

## FIG. 1

| Parameter | Time | Speed | 30 seconds run distance | Accumulative run distance | d-Md | T-MT | $(T-MT)^2$ | (d-Md)*(T-MT) |
|---|---|---|---|---|---|---|---|---|
| | Second | km/hr | second*km/hr | second*km/hr | | | | |
| 0 minute | 0 | 10 | 300 | 300 | | | | |
| | 30 | 10 | 300 | 600 | | | | |
| 1 minute | 60 | 10 | 300 | 900 | | | | |
| | 90 | 10.5 | 315 | 1215 | | | | |
| 2 minutes | 120 | 11 | 330 | 1545 | | | | |
| | 150 | 11 | 330 | 1875 | | | | |
| 3 minutes | 180 | 11 | 330 | 2205 | -2294 | -210 | 44100 | 481740 |
| | 210 | 11 | 330 | 2535 | -1964 | -180 | 32400 | 353520 |
| 4 minutes | 240 | 11 | 330 | 2865 | -1634 | -150 | 22500 | 245100 |
| | 270 | 11 | 330 | 3195 | -1304 | -120 | 14400 | 156480 |
| 5 minutes | 300 | 11 | 330 | 3525 | -974 | -90 | 8100 | 87660 |
| | 330 | 11 | 330 | 3855 | -644 | -60 | 3600 | 38640 |
| 6 minutes | 360 | 11 | 330 | 4185 | -314 | -30 | 900 | 9420 |
| | 390 | 11 | 330 | 4515 | 16 | 0 | 0 | 0 |
| 7 minutes | 420 | 11 | 330 | 4845 | 346 | 30 | 900 | 10380 |
| | 450 | 11 | 330 | 5175 | 676 | 60 | 3600 | 40560 |
| 8 minutes | 480 | 11 | 330 | 5505 | 1006 | 90 | 8100 | 90540 |
| | 510 | 10.5 | 315 | 5820 | 1321 | 120 | 14400 | 158520 |
| 9 minutes | 540 | 10 | 300 | 6120 | 1621 | 150 | 22500 | 243150 |
| | 570 | 10 | 300 | 6420 | 1921 | 180 | 32400 | 345780 |
| 10 minutes | 600 | 10 | 300 | 6720 | 2221 | 210 | 44100 | 466410 |
| Average | 390 | | | 4499 | 0 | 0 | | |
| Total | | | | | | | 252000 | 2727900 |

$$RSV_{HRC} = 10.825 \quad (km/hr)$$

$$RSV_{HRC} = \frac{\Sigma(d-Md)*(T-MT)}{\Sigma(T-MT)^2} = \frac{2727900}{252000} = 10.825 \ (km/hr)$$

FIG. 2

| Parameter | Time | Speed | 30seconds run distance | Accumulative run distance | d-Md | T-MT | $(T-MT)^2$ | $(d-Md)*(T-MT)$ |
|---|---|---|---|---|---|---|---|---|
| | Second | km/hr | 秒*km/hr | second*km/hr | | | | |
| 0 minute | 0 | 9 | 270 | 270 | | | | |
| | 30 | 9 | 270 | 540 | | | | |
| 1 minute | 60 | 9 | 270 | 810 | | | | |
| | 90 | 9.5 | 285 | 1095 | | | | |
| 2 minutes | 120 | 10 | 300 | 1395 | | | | |
| | 150 | 10 | 300 | 1695 | | | | |
| 3 minutes | 180 | 10 | 300 | 1995 | -2011 | -210 | 4044121 | 44100 |
| | 210 | 10 | 300 | 2295 | -1711 | -180 | 2927521 | 32400 |
| 4 minutes | 240 | 10 | 300 | 2595 | -1411 | -150 | 1990921 | 22500 |
| | 270 | 10 | 300 | 2895 | -1111 | -120 | 1234321 | 14400 |
| 5 minutes | 300 | 10 | 300 | 3195 | -811 | -90 | 657721 | 8100 |
| | 330 | 9.5 | 285 | 3480 | -526 | -60 | 276676 | 3600 |
| 6 minutes | 360 | 9.5 | 285 | 3765 | -241 | -30 | 58081 | 900 |
| | 390 | 9.5 | 285 | 4050 | 44 | 0 | 1936 | 0 |
| 7 minutes | 420 | 9 | 270 | 4320 | 314 | 30 | 98596 | 900 |
| | 450 | 9 | 270 | 4590 | 584 | 60 | 341056 | 3600 |
| 8 minutes | 480 | 9 | 270 | 4860 | 854 | 90 | 729316 | 8100 |
| | 510 | 9 | 270 | 5130 | 1124 | 120 | 1263376 | 14400 |
| 9 minutes | 540 | 8.5 | 255 | 5385 | 1379 | 150 | 1901641 | 22500 |
| | 570 | 8.5 | 255 | 5640 | 1634 | 180 | 2669956 | 32400 |
| 10 minutes | 600 | 8.5 | 255 | 5895 | 1889 | 210 | 3568321 | 44100 |
| Average | 390 | | | 4006 | 0 | 0 | | |
| Total | | | | | | | 252000 | 2340900 |

$$RSV_{HRC} = 9.289 \quad (km/hr)$$

$$RSV_{HRC} = \frac{\Sigma(d-Md)*(T-MT)}{\Sigma(T-MT)^2} = \frac{2340900}{252000} = 9.289 \ (km/hr)$$

FIG. 3

**EP 1 808 121 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4566461 A
- US 4112928 A